# EUROPEAN PATENT SPECIFICATION

(11) **EP 3 564 264 B1**
(45) Date of publication and mention of the grant of the patent: **08.09.2021**
(21) Application number: 17840472.9
(22) Date of filing: 20.12.2017
(51) Int. Cl.: C07K 16/24, A61K 39/00

(54) **VACCINE COMPOSITION COMPRISING A MUTANT OF HUMAN INTERLEUKIN-15**
IMPFSTOFF ENTHALTEND EINE IL-15 MUTANTE DES HUMANEN IL-15
VACCINES CONTENANT UN MUTANT IL-15 DE L'INTERLEUKINE-15 HUMAINE

(30) Priority: 30.12.2016 CU 20160194
(43) Date of publication of application: 06.11.2019
(73) Proprietor: Centro De Ingenieria Genetica Y Biotecnologia, La Habana 11600 (CU)
(72) Inventor: RODRIGUEZ ALVAREZ, Yunier, Artemisa 34150 (CU); MORERA DÍAZ, Yanelys, La Habana 13545 (CU); GERONIMO PEREZ, Haydeé, La Habana 11600 (CU)
(74) Representative: Capitán García, Maria Nuria
(86) International application number: PCT/CU2017/050008
(87) International publication number: WO 2018/121802

(56) References cited:
- WO-A2-2006/017853
- VILLADSEN L S ET AL: "Resolution of psoriasis upon blockade of IL-15 biological activity in a xenograft mouse model", JOURNAL OF CLINICAL INVESTIGATION, AMERICAN SOCIETY FOR CLINICAL INVESTIGATION, US, vol. 112, no. 10, 1 November 2003 (2003-11-01), pages 1571-1580, XP002293185, ISSN: 0021-9738, DOI: 10.1172/JCI200318986
- PETTIT D K ET AL: "STRUCTURE-FUNCTION STUDIES OF INTERLEUKIN 15 USING SITE-SPECIFIC MUTAGENESIS, POLYETHYLENE GLYCOL CONJUGATION, AND HOMOLOGY MODELING", JOURNAL OF BIOLOGICAL CHEMISTRY, AMERICAN SOCIETY FOR BIOCHEMISTRY AND MOLECULAR BIOLOGY, US, vol. 272, no. 4, 24 January 1997 (1997-01-24), pages 2312-2318, XP002042891, ISSN: 0021-9258, DOI: 10.1074/JBC.272.4.2312
- FERRARI-LACRAZ SYLVIE ET AL: "Targeting IL-15 receptor-bearing cells with an antagonist mutant IL-15/Fc protein prevents disease development and progression in murine collagen-induced arthritis", THE JOURNAL OF IMMUNOLOGY, THE AMERICAN ASSOCIATION OF IMMUNOLOGISTS, US, vol. 173, no. 9, 1 November 2004 (2004-11-01), pages 5818-5826, XP002345721, ISSN: 0022-1767

## Description

### Field of technique

The present invention relates to the branch of biotechnology, immunology and the pharmaceutical industry, in particular with active immunization using polypeptides comprising a mutated Interleukin-15 (IL-15), for the treatment of diseases related with IL-15 overexpression.

### State of the prior art

The cytokine known as IL-15 is a 14-15 KDa glycoprotein, which was simultaneously described by two groups as a T cells-activating growth factor (Grabstein KH., et al. Science 1994; 264: 965-8; Burton, JD., et al. Proc. Natl. Acad. Sci. USA 1994; 91: 4935-9). IL-15 mRNA is widely expressed in cells and tissues, however, it is difficult to find the protein in these cells or in the cells supernatant due to a strong post-transcriptional control of its expression at the translational level and the intracellular trafficking (Bamford RN., et al. J. Immunol. 1998; 160: 4418-26; Kurys G., et al. J Biol Chem. 2000; 275: 30653-9).

The IL-15 biological effects are mediated through its binding to a cell membrane receptor composed of three subunits : α, β, and γ. The IL-15Rα is a specific, high-affinity (Kd 10⁻¹¹) IL-15 binding subunit, β subunit is shared with IL-2 and γ subunit is a common receptor for several cytokines, such as: IL-2; IL-4; IL-7; IL-9; IL-15; IL-21 (Bamford RN., et al. Proc. Natl. Acad. Sci. USA 1994; 91: 4935-9; Giri JG., et al. EMBO J. 1995; 14: 3654-63).

The IL-15 is an immunostimulatory cytokine that promotes proliferation and functional activity of T, B and NK cells (Giri JG., et al. EMBO J. 1994; 13: 2822-30), activates neutrophils and modifies monokine secretion (Girard D., et al. Blood 1996; 88: 3176-84; Alleva DG., et al. J. Immunol. 1997; 159: 2941-51). Also, this cytokine induces CD56 NK cells proliferation and acts, together with IL-12, inducing IFN-γ and tumor necrosis factor (TNF)-α (Ross ME. and Caligiuri MA. Blood 1997; 89: 910-8; Fehniger TA., et al. Transplant Proc. 1999; 31: 1476-8).

Unregulated expression or overexpression of IL-15 has been associated with the development of several diseases, including Crohn's disease (Kirman I. and Nielsen OH. Am. J. Gastroenterol. 1996; 91(9): 1789-94), psoriasis (Rückert R., et al., J. Immunol. 2000; 165: 2240-50), leukemia (Yamada Y., et al. Blood 1998; 91: 4265-72; Yamada Y. and Kamihira S. Leuk Lymphoma 1999; 35(1-2):37-45) and Rheumatoid arthritis (AR) (McInnes IB. Immunology Today 1998; 19: 75-9).

Previous studies have suggested that IL-15 precede TNF-_{α}, in the cytokine cascade, acting as an important factor in the migration of T cells to synovial fluid (Feldmann M., et al. Annu Rev Immunol. 1996; 14: 397-440; McInnes IB., et al. Nat Med. 1997; 3: 189-95). Additionally, Ziolkowska et al. reported that IL-15 induces IL-17 expression at joints from RA patients and it stimulates release by synoviocytes of several inflammatory mediators such as IL-6, IL-8, GM-CSF, and prostaglandin E₂; sµggesting an important role for IL-15 in the RA pathogenesis (Ziolkowska M., et al. J Immunol. 2000; 164: 2832-8).

The use of IL-15 antagonist molecules has been shown to be effective in animal models. Ruchatz et al. generated a soluble fragment from the alpha subunit of the murine receptor (IL-15Rα) and demonstrated that this fragment inhibited collagen-induced arthritis in DBA/1 mice (Ruchatz H., et al. J. Immunol. 1998; 160: 5654-60).

Other IL-15 antagonist molecules have been patented, including mutants of IL-15 in one or more amino acids residues and monoclonal antibodies against mature IL-15 that prevents signal transduction through its receptor (Patents Number. US6001973, US6177079, US6168783, US6013480). Although, their use is described in the aforementioned patent documents, there are few studies in the literature that support its effectiveness.

On May 2002, Genmab announced the clinical study phase I and II of an anti-IL-15 antibody (HuMax-IL-15) (Patent Number. US6177079). Although, the treatment was well tolerated, no significant efficacy was demonstrated in a phase II study involving 110 patients with RA (Baslund B., et al. Arthritis & Rheumatism. 2005; 52: 2686-92). Other studies have been performed with a chimeric protein consisting of an IL-15 mutant and the Fc fragment of an immunoglobulin, which binds to cells expressing the IL-15 receptor. This fusion protein, called CRB-15, has been shown to be effective in a murine model of collagen induced arthritis, where CRB-15 decreases the incidence and severity of the disease, and prevents the progression of arthritis once established (Ferrari-Lacraz S., et al. J Immunol. 2004; 173(9):5818-26).

In 2003, Santos et al. proposed a vaccination method that included active immunization with IL-15 obtained from *Escherichia coli* (International Patent Application Publication Number WO 2004/032956). However, the protein obtained in that host is biologically active as it promotes the proliferation of the CTLL-2, an IL-2/ IL-15 dependent cell line (Santos-Savio A., et al. Biotecnol. Apl. 2000; 17: 221-4). This element is a disadvantage, taking into account that small amounts of the immunogen that are released and enter into circulation would trigger an unwanted response. This cytokine, under physiological conditions, is known to be a memory T cell growth factor (Kanai T., et al. J Immunol. 1996; 157(8): 3681-7; Kanegane H. and Tosato G. Blood 1996; 88(1): 230-5). The inflammatory process in RA is characterized by activation and recruitment of T cells to the synovial (Wilkinson PC. and Liew FY. Exp Med. 1995; 181: 1255-9), so immunization with an active IL-15 molecule may contributes to the development of the disease.

In "Resolution of psoriasis upon blockade of IL-15 biological activity in a xenograft mouse model" (Louise S. Villadsen, et al. Published November 15, 2003, J Clin Invest. 2003;112(10):1571-1580. https://doi.org/10.1172/JCI18986.", using a human antibody against IL-15, it was collected evidence for a prominent role of IL-15 in the pathogenesis of psoriasis. Herein in vitro and in vivo studies indicate that mAb 146B7 is able to inhibit IL-15-induced T cell proliferation, inhibit production of IL-15-induced TNF-α production, reverse IL-15-mediated protection against apoptosis of monocytes, and decrease the number of inflammatory cells in the psoriatic lesion. WO2006017853 discloses methods of treating patients who have received, or who are scheduled to receive, a heart, lung, or heart-lung transplant by administering to the patient an agent that antagonizes IL-15 receptor (IL-15R).

In "Structure-Function Studies of Interleukin 15 using Site-specific Mutagenesis, Polyethylene Glycol Conjugation, and Homology Modeling" (Dean K. Pettit et al., The Journal of Biological Chemistry 272, 2312-2318 doi: 10.1074/jbc.272.4.2312, January 24, 1997), recombinant human IL-15 was PEGylated via lysine-specific conjugation chemistry in order to extend the circulation half-life of this cytokine. Specifically, PEG-IL-15 lost its ability to stimulate the proliferation of CTLL but took on the properties of a specific IL-15 antagonist in vitro. In comparing sequence alignments and molecular models for IL-2 and IL-15, it was noted that lysine residues resided in regions of IL-15 that may have selectively disrupted receptor subunit binding. We hypothesized that PEGylation of IL-15 interferes with β but not α receptor subunit binding, resulting in the IL-15 antagonist activity observed in vitro.

The validity of this hypothesis was tested by engineering site-specific mutants of human IL-15 as suggested by the IL-15 model (IL-15D8S and IL-15Q108S block β and γ receptor subunit binding, respectively). As with PEG-IL-15, these mutants were unable to stimulate CTLL proliferation but were able to specifically inhibit the proliferation of CTLL in response to unmodified IL-15.

It remains of interest to obtain vaccines for active immunotherapy in diseases related with IL-15 overexpression, which offer therapeutic advantages on current therapies aimed at the treatment of this type of diseases.

### DETAILED DESCRIPTION OF THE INVENTION

The present invention solves the above-mentioned problem by providing a vaccine composition characterized in that it contains a polypeptide which is a mutant of the human IL-15 identified as SEQ ID No. 1, at least one vaccine adjuvant and pharmaceutically acceptable excipients or carriers.

The IL-15 gene was isolated from lipopolysaccharide (LPS)-activated monocytes and amplified by polymerase chain reaction (PCR), using oligonucleotides specific for mutation in the sequence of IL-15. Specifically, the Asp 8 and Gln 108 were replaced by Ser. The mutated polypeptide was obtained with the modifications in the amino acid residues mentioned above, hereinafter referred to as IL-15Mut, having a purity of greater than 95%.

In one embodiment of the invention, the vaccine composition comprises a polypeptide containing the identified IL-15 mutant (IL-15Mut) SEQ ID No. 1, which is obtained by recombinant deoxyribonucleic acid (DNA) technology. To obtain this molecule, hosts which are known to those skilled in the art, such as bacteria, yeasts, mammalian cells, etc. may be employed.

In one embodiment of the invention, the IL-15Mut polypeptide was obtained from *E. coli,* so it is not glycosylated, unlike the autologous protein present in the human. This mutated cytokine is not biologically active in the cell proliferation assay in CTLL-2, since it is not able to induce the growth of these cells, which are dependent on IL-15. In CTLL-2 cells, IL-15Mut competes with native IL-15 for receptor binding, but does not induce signaling by having mutated the amino acids Asp 8 and Gln 108, which are key residues for the interaction with the β and γ subunits of the receptor. This element is an advantage over the method proposed by Santos et al. (International Patent Application Publication No. WO 2004/032956), regarding a lower occurrence of adverse events, with respect to the pro-inflammatory activity of this cytokine, in the treated patients.

The polypeptide which is the IL-15 mutant identified as SEQ ID No. 1, which is part of a vaccine composition, is a fusion polypeptide. The fusion polypeptide which is the IL-15 mutant identified as SEQ ID No. 1 also comprises T epitopes, at a protein concentration of 200 µg.

In the present invention, it was designed an IL-15 mutant which corresponds to SEQ ID No. 1 obtaining by recombinant DNA technology; with structural characteristics different from the natural cytokine with respect to the localization of the disulfide bridges. This element promotes the development of the immune response by exposure of cryptic or immunodominant epitopes.

To demonstrate the concept proposed in this invention non-human primates (*Chlorocebus sabaeus*) were used; taking into account that this is the species with the highest homology, in the amino acid sequence of IL-15, with respects to human (∼97% of identity) (Grabstein KH., et al. Science 1994; 264(5161):965-8). It was demonstrated that active immunization of such primates, with a vaccine composition comprising the IL-15Mut polypeptide identified as SEQ ID No. 1, using aluminum hydroxide (alternatively called Alumina) and the oily adjuvant Montanide ™ ISA-51, elicits a response of antibodies that inhibit the biological activity of native IL-15 in CTLL-2 cells. This cell line, used for the neutralization assays, expresses the three subunits of the IL-15 receptor, and is dependent on this cytokine to proliferate (Eisenman J., et al. Cytokine 2002; 20(3): 121-9). In this assay, the sera showed inhibition the proliferation in a dose-dependent manner. This response is higher when compared to the group of animals immunized with the polypeptide identified as SEQ ID No. 2 (IL-15 protein obtained in *E. coli*)*.* The results also shown that immunization with a higher dose (350 µg) of the polypeptide of SEQ ID No.1, generates a higher neutralizing antibody response from the 15 days after the second immunization. This aspect is an advantage, being that the desired effect is achieved with a lower number of administrations of the vaccine composition of the present invention.

Considering the high percentage of similarity between the mutated polypeptide of SEQ ID No. 1 and simian IL-15, it is unexpected that immunization with this mutated protein generates a neutralizing antibody response against native IL-15. However, the present invention demonstrates that immunization with the vaccine according to claim 1 including the mutated polypeptide identified as SEQ ID No. 1 generates neutralizing autoantibodies against autologous IL-15. In addition, it is surprising that the neutralizing antibody response generated by administration of that vaccine including the polypeptide (SEQ ID No. 1) is greater than the obtained by active immunization with recombinant IL-15 (SEQ ID No. 2).

The vaccine composition of the present invention may be administered by several routes, as it is known to those skilled in the art. The amount of vaccine antigen in the composition may vary, depending on the disease to be treated. In one embodiment of the invention, the amount of antigen (polypeptide comprising a mutant of the human IL-15 identified as SEQ ID No. 1) is in the range of 200-500 µg per vaccine dose. In the course of patient immunization, repeated doses may be administered and the compositions may contain vaccine adjuvants which are well known in the current vaccination, such as aluminum salts and water-in-oil emulsions, and others which are in development; in order to generate in the patient the levels of antibodies against IL-15 that are required.

The invention also discloses the use of a polypeptide which is a mutant of human IL-15, identified as SEQ ID No. 1, to manufacture a medicament for the active immunotherapy of a disease related with IL-15 overexpression. It is well known to those skilled in this field of the art those diseases involving aberrant expression or over-expression of IL-15.

The polypeptide which is a human IL-15 mutant identified as SEQ ID No. 1, is used to manufacture a vaccine for the active immunotherapy of an autoimmune disease selected from the group consisting of the RA, Crohn's disease, ulcerative colitis and psoriasis.

The vaccine is used for the active immunotherapy of a disease related with IL-15 overexpression, such as hematological malignancies. In a particular embodiment, the hematological neoplasia is leukemia or a lymphoma.

It is also disclosed a method for the treatment of diseases related with IL-15 overexpression. It is characterized by comprising the administration to an individual that needs it of a therapeutically effective amount of a vaccine composition that comprises a polypeptide which is a mutant of the human IL-15 identified as SEQ ID No. 1. As stated above, said vaccine composition is suitable for administration to humans, and generates a neutralizing antibody response against autologous IL-15.

The method comprising active immunization with the IL-15Mut (of SEQ ID No. 1) in a formulation suitable to generate neutralizing autoantibodies against IL-15, enables the neutralization of unregulated amounts of this cytokine in patients with autoimmune diseases and hematological malignancies. This method is superior to that proposed in the International Patent Application No. WO 2004/032956, taking into account that the antigen used for the active immunization lacks biological activity.

The method is useful for the treatment of diseases related with IL-15 overexpression, including RA, psoriasis and inflammatory bowel diseases. Particularly in these pathologies, the generation of antibodies that neutralize the IL-15 activity is advantageous. In patients affected by these diseases, the active immunotherapy method of the invention may be combined with the administration of other medicaments, such as anti-inflammatories or antagonists of other cytokines. These medicaments include, for example, steroid drugs, such as corticosteroids and traditional disease-modifying antirheumatic drugs (e.g. methotrexate).

The method is also useful for the treatment of diseases such as hematological malignancies, in which leukemia and lymphomas are found. In this context, the active immunotherapy employing the vaccine composition of the invention may be combined with the administration, to the individual requiring it, of at least one pharmaceutically acceptable cytostatic.

The method is advantageous, since the levels of antibodies generated in the patient itself are more stable over time than those administered by passive immunization. On the other hand, the frequency of application of the vaccine compositions comprising the IL-15 mutant is much less than in a passive immunization with antibodies against the cytokine (in the method of this invention they are spaced for a longer time interval). In addition, the amounts of protein per dose, and the amount of administrations required for vaccination, are lower than those required for treatment with other IL-15 antagonist molecules, which implies a lower cost of production and higher adherence to treatment.

Immunization with the vaccine according to claim 1 including the polypeptide identified as SEQ ID No. 1 generates antibodies with a greater neutralizing capacity than those obtained in the group immunized with the recombinant protein of SEQ ID No. 2. The use of an antigen that is not biologically active results in a lower frequency of undesirable events in patients treated with the vaccine containing it.

### Brief Drawings Description

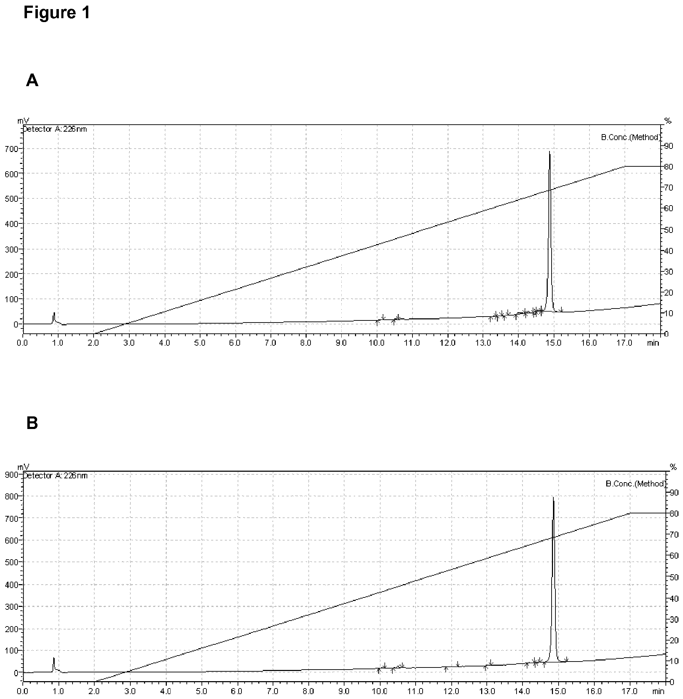

**Figure 1****.** Purity of IL-15Mut. RP-HPLC chromatographic profile of IL-15Mut (A) and recombinant IL-15 (B) obtained in *E. coli,* used as antigen of the vaccine preparation.

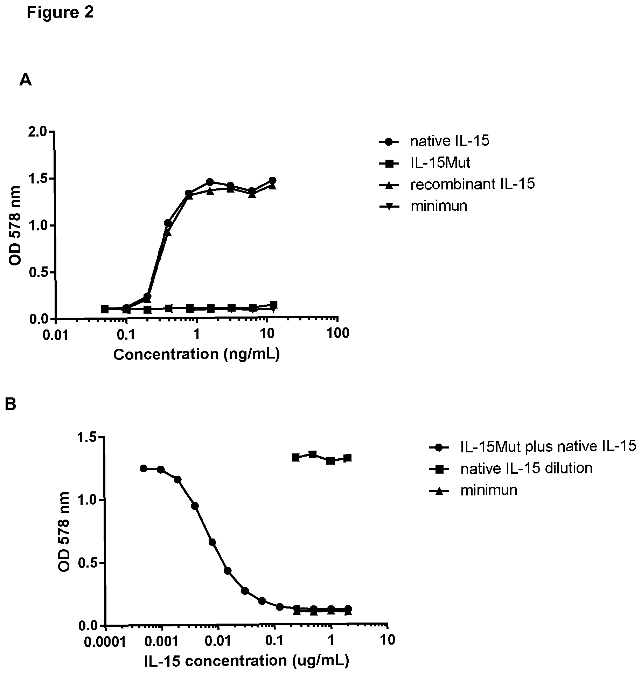

**Figure 2****.** Evaluation of IL-15Mut biological activity by CTLL-2 cell proliferation assay. (A) Cell proliferation induced by serial dilutions of native IL-15 (R&D), IL-15Mut and recombinant IL-15 obtained in *E. coli.* (B) Cell proliferation curve in the presence of serial dilutions of IL-15Mut with a fixed concentration of native IL-15.

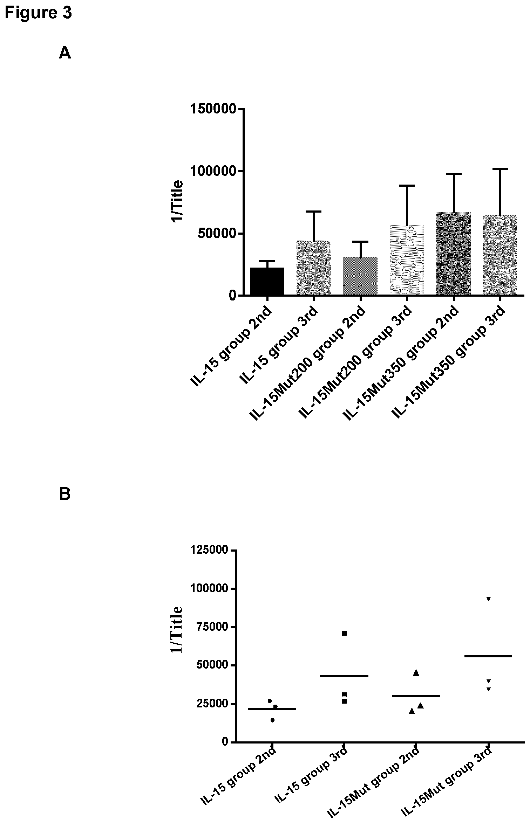

**Figure 3****.** Evaluation by ELISA of the anti-IL-15 antibody response in monkeys immunized with IL-15Mut or IL-15, using Aluminum hydroxide (A) or Montanide ™ ISA-51 (B) as adjuvant. The graphs represent the mean values of the antibody titers of the three animals in each experimental group, corresponding to 15 days after the second and the third immunization.

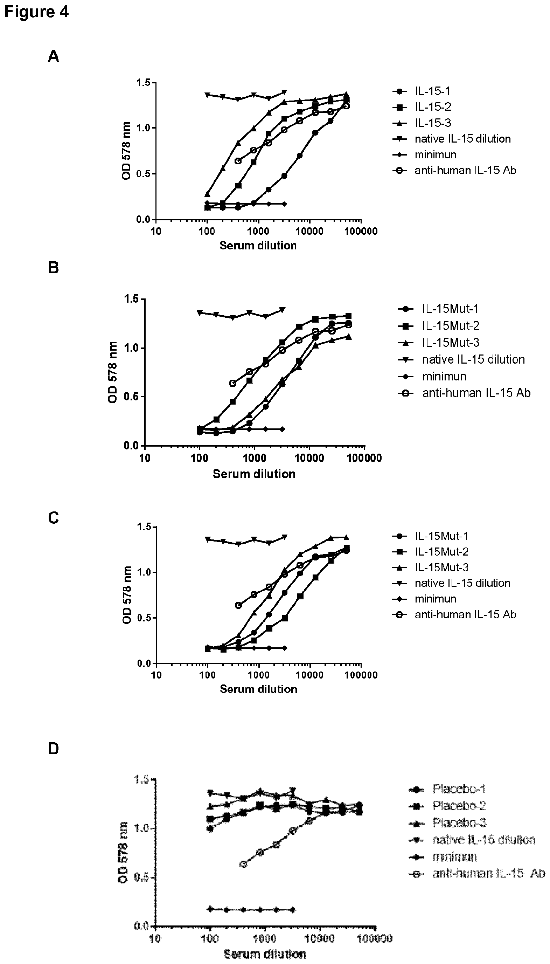

**Figure 4****.** Evaluation in CTLL-2 cells of the neutralizing capacity of the individual sera after the third immunization using Alum as adjuvant. (A) IL-15 group, (B) IL-15Mut group at dose of 200 µg, (C) IL-15Mut group at dose of 350 µg, and (D) placebo group. The line called native IL-15 dilution represents the highest value of cell proliferation and the line named minimum indicates the minimum proliferation value for CTLL-2 cells grown in cytokine-free culture medium.

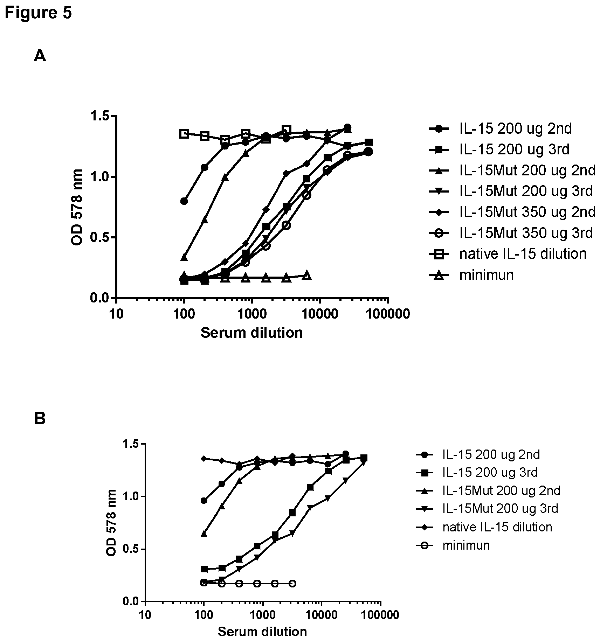

**Figure 5****.** Evaluation in CTLL-2 cells of the neutralizing capacity of the pool of sera from animals immunized with IL-15Mut and recombinant IL-15. The neutralizing effects of each group, 15 days after the second and third immunizations, using aluminum hydroxide (A) or Montanide ™ (B) as adjuvant are shown. The line called native IL-15 dilution represents the highest value of cell proliferation and the line named minimum indicates the minimum proliferation value for CTLL-2 cells grown in cytokine-free culture medium.

### Detailed Disclosure of Embodiments/ Examples of realization

### Example 1. Obtaining polypeptide IL-15Mut in E. coli

DNA coding for human IL-15 was isolated from LPS-activated monocytes and it was amplified by PCR (annealing temperature 60°C for 25 cycles), using specific primers for the mutation of Asp⁸ and Gln¹⁰⁸ by Ser in the IL-15 sequence (primer 5' CAT GCC ATG GCA AAC TGG GTG AATGTA ATA AGT TCT TTG AAA and primer 3' C GGGATCCCG TTA AGA AGT GTT GAT GAA CAT AGA GAC AAT). The PCR product was digested with Nco I/BamH I (Promega, USA) and cloned into an *E.coli* expression vector. The IL-15Mut, obtained as inclusion bodies, was solubilized with urea 8M. The renaturation process was carried out on a 1.6 x 40 cm column (GE Healthcare Life Sciences, USA) packed with Sephadex G-25 Fine (Pharmacia Biotech, Sweden) and equilibrated with 0.1 M Tris buffer and 0,15 M NaCl; pH 8.0 at the rate flow of 7 mL/min. The collected sample was applied to a 1.6x10cm column (GE Healthcare, USA) packed with Q Sepharose Fast Flow (GE Healthcare, USA), using a linear gradient of elution from 0.2 M to 0.5 M NaCl, at a flow rate of 2 mL/min. The sample eluted with 0.5 M NaCl was applied to a C₄ column (1 × 25 cm, 10 µm, Vydac, USA), at a flow of 1.0 mL/min. Proteins were separated using a mobile phase containing 0.1% trifluoroacetic acid (TFA) and acetonitrile HPLC grade); employing the following gradient: 0-30% in 5 min; 30% for 10 min; 30-40% in 5 min; 40-50% in 30 min; 50-60% in 60 min; 60-80% in 5 min at a flow of 2.5 mL/min. Proteins separation were monitored at 226 nm. The Bradford method was employed to determine the total protein concentration, according to manufacturer's instructions. Non-mutated IL-15 protein was also obtained recombinantly in *E. coli,* as previously described (Santos-Savio A., et al. Biotecnol. Apl. 2000; 17: 221-4).

### Example 2. Determination of IL-15Mut purity by RP-HPLC

Samples collected from the major RP-HPLC purification peak were checked to estimate percent purity. The protein concentration was determined by the modified Lowry method, with the set of reagents *"Modified Lowry Protein Assay Kit*" (Thermo Scientific, USA), according to manufacturer's instructions. The RP-HPLC analysis was conducted with 50 µg of purified proteins on Chromolith Performance C₈ column (4.6 × 100 mm, 2 µm, Merck, USA) using a gradient from 0 to 80% of AcN/0.1% TFA in 15 min, at a flow rate of 2.5 mL/min. The detection wavelength was set at 226 nm. The purity was determined using the program Image J v1.32. IL-15Mut was obtained with a purity of 96%, as seen in Figure 1; while non-mutated recombinant IL-15 was obtained with 98% of purity.

### Example 3. Evaluation of the IL-15Mut biological activity in CTLL-2 cell line

In order to evaluate the biological activity of IL-15Mut, the proliferation assay in CTLL-2 cell line was performed. Biological activity was measured by stimulation of CTLL-2 cells proliferation, using mitochondrial staining with 3- (4,5-dimethylthiazol-2-yl) -2,5-diphenyltetrazolium bromide (MTT, 3- [4, 5-Dimethylthiazol-2-yl] -2,5-diphenyltetrazolium bromide) (Mossman TJ. Immunol. Methods 1983; 65(1-2): 55-63), following the procedure described below. Twofold serial dilutions of recombinant IL-15, native IL-15 (R&D, USA) and IL-15Mut (starting concentration 25 ng/mL) were performed in 96-well plates (Costar, USA) in a volume of 50 µL of RPMI medium supplemented with 10 % of fetal bovine serum (FBS) and 50 µg/mL of gentamycin. In addition, serial dilutions of the mutated IL-15 from 6 µg/mL, were performed in 30 µL of supplemented RPMI medium. Dilutions of IL-15Mut were co-incubated with 20 µL of 300 pg/mL native IL-15. Then, previously washed CTLL-2 cells with RPMI medium were added in amounts of 5 × 10³ cells/well in 50 µL. The plate was incubated for 72 h at 5% CO₂, 37°C and 98% relative humidity. Cell viability was determined by MTT staining. IL-15Mut was not biologically active, as it did not induce the proliferation of CTLL-2 cells, unlike recombinant IL-15 (SEQ ID No. 2) and native IL-15, which stimulate the proliferation in a dose-dependent manner (Figure 2).

### Example 4. Immunization scheme in green monkeys

The animals (green monkeys *Chlorocebus sabaeus*) were divided into 6 groups of 3 animals each one. A placebo group which received saline phosphate buffer (PBS) solution in aluminum hydroxide adjuvant (Brenntag Biosector, Denmark), a group immunized with 200 µg of the polypeptide identified as SEQ ID No. 2 (IL-15 group), and two other groups, which received 200 µg or 350 µg of the polypeptide identified as SEQ ID No. 1 combined with aluminum hydroxide at a concentration of 1.8 mg/ mL. In addition, two other experimental groups immunized with 200 µg of the polypeptide identified as SEQ ID No. 1 or SEQ ID No. 2 in Montanide ™ ISA-51 adjuvant (50:50 v/v) were included. Administration of the immunogen was performed subcutaneously at several sites of the interscapular region in a total volume of 0.5 mL. Three immunizations were performed, spaced 15 days between the first and second, and 2 months between the second and third. Blood samples were collected before beginning the scheme (pre-immune serum) and 15 days after the second and third immunization. Complement was inactivated by incubating the sera at 56°C for 30 min. Vital signs, temperature, heart rate and body weight were registered along the whole scheme.

### Example 5. Determination of anti-IL-15 antibodies in serum from immunized animals.

Specific Abs titers against IL-15 were measured through an indirect ELISA, where EIA 96-well plates (Costar, USA) were coated for 16 h at 25°C with 1 µg/mL of polypeptide identified as SEQ ID No. 2. The plates were blocked with 1% bovine serum albumin (BSA, Sigma-Aldrich, USA) in PBS for 90 min at 37 °C followed by 3 washes with 0.05% Tween 20 in PBS. PBS, 0.05% Tween 20 and 0.01% BSA-diluted sera (starting dilution 1:5000) were added to wells in a volume of 100 µL and incubated for 90 min at 37°C. Wells were then washed 3 times and incubated with anti-human IgG (Fc specific)-peroxidase Ab (Sigma, USA) diluted 1: 10 000 in PBS. After incubating for 1 h at 37 °C, the plates were washed 5 times and incubated with 100 µL of substrate solution. The reaction was stopped at 15 min by adding 50 µL of 0.5 M sulphuric acid solution and the absorbance at 450 nm was measured with an ELISA plate reader. Ab titer was considered as the highest serum dilution yielding at least twice the value of the optical density (OD) at 450 nm of the pre-immune serum from each animal. The data were processed using the GraphPad Prism program v6.05 (GraphPad Software, Inc.). The results obtained are shown in Figure 3; which indicates that active immunization with the mutated polypeptide of IL-15 (corresponding to SEQ ID No. 1) generates an anti-IL-15 antibody response both when using alumina or Montanide ™ as adjuvant.

### Example 6. Determination of the neutralizing capacity of sera from immunized animals

To evaluate the neutralizing capacity of sera the CTLL-2 cell proliferation assay was performed. This IL-2-dependent cell line also proliferates in presence of IL-15 (maximum proliferation corresponds to cells incubated with 300 pg/mL of native IL-15), whereas in the absence of the cytokine the cells do not proliferate (minimum: cells grown in culture medium without IL-15). In this assay, the molecules that bind to IL-15 and impede the signal translation through its receptor are detected because they inhibit the proliferation of this cell line.

To evaluate the neutralizing capacity of antibodies from immune monkeys, twofold serial dilutions of heat-inactivated sera (starting dilution 1:100) were performed in 96-well plates in a volume of 30 µL of RPMI medium supplemented with 10% FBS and 50 µg/mL of gentamycin. Then, 300 pg/mL of native human IL-15 (R&D, USA) in a volume of 20 µL was added to each well and the plate was incubated at 37°C for 30 min. Afterwards, previously washed CTLL-2 cells were added in amounts of 5 × 10³ cells/well in 50 µL and the plate was incubated for 72 h at 5% CO₂, 37°C and 98% of relative humidity (Rodríguez Y., et al. Biotecnol. Apl. 2014; 31: 291-6). MAB2471(R&D, USA), a neutralizing anti-human IL-15 Ab, was used as a positive control at starting concentration of 1 µg/mL (twofold serial dilutions). Cell viability was determined by MTT staining. The neutralizing titers of sera were expressed as the dilution of sera that is required for inhibiting the proliferation by at least 50% (ID₅₀) and were calculated using the Curve Expert Program version 1.3.8.0. Figure 4 shows the inhibitory effect of serum on native IL-15-induced proliferation in CTLL-2 cells. These sera were isolated from animals immunized with the polypeptide of SEQ ID No. 1 (IL-15Mut) and SEQ ID No. 2 (recombinant IL-15) using aluminum hydroxide as adjuvant. ID₅₀ values of serum from the individual animals within each experimental group (3 animals per group) are shown in Table 1. When comparing the neutralization titers of the animals receiving the same dose (200 µg) of IL-15Mut (SEQ ID No. 1) and recombinant IL-15 (SEQ ID No. 2), they were found to be higher for the mutated cytokine.

**Table 1. ID₅₀ values of serum from animals corresponding to 15 days after the third immunization.**

| **Immunogen/Dose** | **Animals** | **Neutralization Titers (ID₅₀)** |
|---|---|---|
| IL-15/200 µg | 1 | 1946 |
| IL-15/200 µg | 2 | 859 |
| IL-15/200 µg | 3 | 280 |
| | | |
| IL-15Mut/200 µg | 1 | 3547 |
| IL-15Mut/200 µg | 2 | 809 |
| IL-15Mut/200 µg | 3 | 3238 |
| | | |
| IL-15Mut/350 µg | 1 | 2273 |
| IL-15Mut/350 µg | 2 | 5218 |
| IL-15Mut/350 µg | 3 | 1171 |

Figure 5 shows the neutralizing capacity in CTLL-2 cells of pool of sera from animals immunized with IL-15Mut and recombinant IL15. The evaluated sera correspond to 15 days after the second and third immunization, using aluminum hydroxide or Montanide ™ as adjuvant. It is observed a neutralizing effect of sera after the third immunization in animals immunized with 200 µg of IL-15Mut and recombinant IL-15. However, the sera corresponding to the dose of 350 µg showed a neutralizing effect at 15 days after the second administration.

Table 2 summarizes the ID₅₀ values of the pool of sera corresponding to 15 days after the second and third immunization, showing the neutralizing titers for the three experimental groups. The immunization with a higher dose (350 µg) of IL-15Mut (SEQ ID No. 1) generates a greater neutralizing response at 15 days after the second immunization, when alumina is used as adjuvant. This is an advantage taking into account that the desired effect could be achieved with fewer administrations. The dose of 200 µg was administrated in combination with Montanide as adjuvant. In this case, the ID₅₀ values for IL-15Mut group are also higher than those achieved for recombinant IL-15 group after the second and third inoculations.

**Table 2. ID₅₀ values of the pool of sera corresponding to 15 days after the second and third immunization.**

| **Immunogen/Dose** | **Number of immunizations** | **Adjuvant** | **Neutralization titers (ID₅₀)** |
|---|---|---|---|
| IL-15/200 µg | 2 | Aluminum hydroxide | 80 |
| IL-15Mut/200 µg | 2 | Aluminum hydroxide | 206 |
| IL-15Mut/350 µg | 2 | Aluminum hydroxide | 1308 |
| IL-15/200 µg | 3 | Aluminum hydroxide | 1256 |
| IL-15Mut/200 µg | 3 | Aluminum hydroxide | 2836 |
| IL-15Mut/350 µg | 3 | Aluminum hydroxide | 3576 |
| IL-15/200 µg | 2 | Montanide™ ISA-51 | 50 |
| IL-15Mut/200 µg | 2 | Montanide™ ISA-51 | 175 |
| IL-15/200 µg | 3 | Montanide™ ISA-51 | 1154 |
| IL-15Mut/200 µg | 3 | Montanide™ ISA-51 | 1952 |

## Claims

1. A vaccine composition **characterized in that** it contains a polypeptide which is a mutant of the human Interleukin-15 (IL-15) consisting of SEQ ID No.1, at least one vaccine adjuvant selected between aluminum salts or water-in-oil emulsions and pharmaceutically acceptable excipients or carriers.

2. The vaccine composition according to claim 1, for use to treat diseases related with IL-15 overexpression selected from autoimmune diseases or a hematologic neoplasia.

3. The vaccine composition for use according to claim 2, wherein the autoimmune diseases are selected from the group consisting of rheumatoid arthritis (RA), Crohn's disease, ulcerative colitis and psoriasis.

4. The vaccine composition for use according to claim 2, wherein the hematologic neoplasia is a leukemia or lymphoma.

5. The vaccine composition for use according to claim 2, wherein it is additionally combined with anti-inflammatory drugs or antagonists of other cytokines.

6. The vaccine composition for use according to claim 2, wherein it is additionally combined with a pharmaceutically acceptable cytostatic.

## Patentansprüche

1. Impfstoffzusammensetzung, **dadurch gekennzeichnet, dass** sie ein Polypeptid enthält, das eine Mutante des humanen Interleukin-15 (IL-15) ist, bestehend aus SEQ ID No.1, mindestens einem Impfstoffadjuvans, ausgewählt aus Aluminiumsalzen oder Wasser-in-ÖI-Emulsionen, und pharmazeutisch unbedenklichen Hilfsstoffen oder Trägern.

2. Impfstoffzusammensetzung nach Anspruch 1 zur Verwendung zum Behandeln von Krankheiten, die mit IL-15-Überexpression zusammenhängen, ausgewählt aus Autoimmunerkrankungen oder einer hämatologischen Neoplasie.

3. Impfstoffzusammensetzung zur Verwendung nach Anspruch 2, wobei die Autoimmunkrankheiten ausgewählt sind aus der Gruppe bestehend aus rheumatoider Arthritis (RA), Morbus Crohn, Colitis ulcerosa und Psoriasis.

4. Impfstoffzusammensetzung zur Verwendung nach Anspruch 2, wobei die hämatologische Neoplasie eine Leukämie oder ein Lymphom ist.

5. Impfstoffzusammensetzung zur Verwendung nach Anspruch 2, wobei sie zusätzlich mit entzündungshemmenden Medikamenten oder Antagonisten anderer Cytokine kombiniert ist.

6. Impfstoffzusammensetzung zur Verwendung nach Anspruch 2, wobei sie zusätzlich mit einem pharmazeutisch unbedenklichen Cytostatikum kombiniert ist.

## Revendications

1. Composition de vaccin **caractérisée en ce qu'**elle contient un polypeptide qui est un mutant de l'interleukine-15 (IL-15) humaine constituée de SEQ ID NO:1, d'au moins un adjuvant de vaccin choisi entre les sels d'aluminium ou les émulsions eau-dans-huile et des excipients ou véhicules pharmaceutiquement acceptables.

2. Composition de vaccin selon la revendication 1, destinée à être utilisée pour traiter des maladies liées à la surexpression de l'IL-15 choisies parmi les maladies auto-immunes ou la néoplasie hématologique.

3. Composition de vaccin destinée à être utilisée selon la revendication 2, les maladies auto-immunes étant choisies dans le groupe constitué de la polyarthrite rhumatoïde (PR), de la maladie de Crohn, de la rectocolite hémorragique et du psoriasis.

4. Composition de vaccin destinée à être utilisée selon la revendication 2, la néoplasie hématologique étant une leucémie ou un lymphome.

5. Composition de vaccin destinée à être utilisée selon la revendication 2, laquelle est de plus associée à des médicaments anti-inflammatoires ou à des antagonistes d'autres cytokines.

6. Composition de vaccin destinée à être utilisée selon la revendication 2, laquelle est de plus associée à un cytostatique pharmaceutiquement acceptable.


### SEQUENCES LIST

<110> CENTER OF GENETIC ENGINEERING AND BIOTECHNOLOGY
<120> VACCINE COMPOSITION COMPRISING A MUTANT OF THE HUMAN INTERLEUKIN-15
<130> Mutated IL-15 vaccine
<140> CU 2016-0194
   <141> 30.12.2016
<160> 4
<170> PatentIn Ver. 2.1
<210> 1
   <211> 114
   <212> PRT
   <213> Artificial Sequence
<220>
   <223> Description of the Artificial Sequence:Human Interleukin-15 Mutant
<400> 1
   Asn Trp Val Asn Val Ile Ser Ser Leu Lys Lys Ile Glu Asp Leu Ile
     1               5                  10                  15
   Gln Ser Met His Ile Asp Ala Thr Leu Tyr Thr Glu Ser Asp Val His
                20                  25                  30
   Pro Ser Cys Lys Val Thr Ala Met Lys Cys Phe Leu Leu Glu Leu Gln
            35                  40                  45
   Val Ile Ser Leu Glu Ser Gly Asp Ala Ser Ile His Asp Thr Val Glu
        50                  55                  60
   Asn Leu Ile Ile Leu Ala Asn Asn Ser Leu Ser Ser Asn Gly Asn Val
    65                  70                  75                  80
   Thr Glu Ser Gly Cys Lys Glu Cys Glu Glu Leu Glu Glu Lys Asn Ile
                    85                  90                  95
   Lys Glu Phe Leu Gln Ser Phe Val His Ile Val Ser Met Phe Ile Asn
               100                 105                 110
   Thr Ser
<210> 2
   <211> 114
   <212> PRT
   <213> Homo sapiens
<400> 2
   Asn Trp Val Asn Val Ile Ser Asp Leu Lys Lys Ile Glu Asp Leu Ile
     1               5                  10                  15
   Gln Ser Met His Ile Asp Ala Thr Leu Tyr Thr Glu Ser Asp Val His
               20                  25                  30
   Pro Ser Cys Lys Val Thr Ala Met Lys Cys Phe Leu Leu Glu Leu Gln
           35                  40                  45
   Val Ile Ser Leu Glu Ser Gly Asp Ala Ser Ile His Asp Thr Val Glu
        50                  55                  60
   Asn Leu Ile Ile Leu Ala Asn Asn Ser Leu Ser Ser Asn Gly Asn Val
    65                  70                  75                  80
   Thr Glu Ser Gly Cys Lys Glu Cys Glu Glu Leu Glu Glu Lys Asn Ile
                    85                  90                  95
   Lys Glu Phe Leu Gln Ser Phe Val His Ile Val Gln Met Phe Ile Asn
               100                 105                 110
   Thr Ser
<210> 3
   <211> 42
   <212> DNA
   <213> Artificial Sequence
<220>
   <223> Description of the sequence Artificial:5'oligonucleotide for obtaining Interleukin-15 mutant
<400> 3
   catgccatgg caaactgggt gaatgtaata agttctttga aa 42
<210> 4
   <211> 40
   <212> ADN
   <213> Artificial Sequence
<220>
   <223> Description of the sequence Artificial:3'oligonucleotide for obtaining Interleukin-15 mutant
<400> 4